# EUROPÄISCHE PATENTANMELDUNG

(11) **EP 1 277 868 A1**
(43) Veröffentlichungstag der Anmeldung: **22.01.2003**
(21) Anmeldenummer: 02011546.5
(22) Anmeldetag: 24.05.2002
(51) Int. Cl.: D04H 13/00, B32B 27/12, B32B 5/26, B32B 3/26, B32B 3/12, B32B 3/28, B32B 31/00

(54) **Komposit-Vliesstoff mit hoher Querfestigkeit, Verfahren zu dessen Herstellung und dessen Verwendung**

(30) Priorität: 19.07.2001 DE 10135111
(71) Anmelder: Carl Freudenberg KG, 69469 Weinheim (DE)
(72) Erfinder: Grimm, Hansjörg, 69469 Weinheim (DE); Groitzsch, Dieter, Dr., 69493 Hirschberg (DE); Pehr, Marc, 69514 Laudenbach (DE)

(57) **Zusammenfassung**

Beschrieben wird ein Verbundstoff umfassend mindestens einen Vliesstoff und ein damit thermisch verbundenes, in Form von regelmäßig wiederkehrenden Mustern angeordnetes Verstärkungsmaterial, das sich von einer Folie aus thermoplastischem Material ableitet.

Der neue Verbundstoff kann als Operationskittel, textiles Obermaterial für den Einmalgebrauch, als Gürtel oder in absorbierenden Produkten, wie Windeln oder Damenhygiene-Produkten, eingesetzt werden.

## Beschreibung

### Technisches Gebiet

Die vorliegende Erfindung betrifft einen neuen Vliesstoffverbund, der sich insbesondere als Operationskittel, textiles Obermaterial für den Einmalgebrauch, als Gürtel oder in absorbierenden Produkten einsetzen lässt.

### Stand der Technik

Es ist allgemein bekannt, dass insbesondere Stapelfaservliesstoffe oft den Nachteil niedriger Festigkeiten quer zur Maschinenlaufrichtung aufweisen, da ihre Fasern mehr oder weniger stark in Maschinenlaufrichtung ausgerichtet sind. Solche starke Längsorientierungen treten dann auf, wenn die Krempeln über den Faserablegebändern positioniert sind, was heute in den meisten Stapelfaser-Vliesstofffertigungen der Fall ist. In den Anwendungsfällen, wo eine möglichst isotrope Faserverteilung bzw. in beiden Richtungen hohe mechanische Festigkeiten verlangt werden, sind solche Stapelfaservliesstoffe von Nachteil, deren Längs-/Querverhältnisse der Höchstzugkraft im Bereich von etwa 3:1 bis 10:1 liegt. Ein Ausweichen auf konventionelle Spinnvliesstoffe, die bekanntermaßen ausgeglichenere Längs- / Querverhältnisse der Höchstzugkraft von ca. 1:1 bis ca. 2:1 aufweisen, je nach Art oder Modifikation des Spinnvliesverfahrens, kann in den meisten Fällen nicht akzeptiert werden, da mit diesen Endlosfilament-Vliesstoffen in der Regel keine vergleichbare Haptik oder Textilität erreicht werden kann.

Die an sich bekannten Methoden der Binderapplikation ändern im Prinzip nichts an dem Längs/Querverhältnis der Höchstzugkraft von Vliesstoffen, selbst wenn nicht ganzflächige Applikationsmethoden sondern Druckmethoden mit querorientiertem Druckmuster angewendet werden. Es werden damit selbstverständlich Festigkeitserhöhungen erzielt. Die Erhöhungen sind jedoch im wesentlichen bezogen auf die Längs- und Querfestigkeiten prozentual die gleichen, so dass sich das Längs / Querverhältnis dadurch nicht ändert. Auch bei Anwendung von ganzflächigen Glatt- oder musterartigen Präge-Kalanderbindungen durch Hitze und Druck bzw. durch Ultraschall lassen sich keine günstigeren Verhältnisse bezüglich der Längs-/Querfestigkeiten erzeugen.

Es ist bekannt, dass Vliesstoffe mit Netzen, Geweben, Gewirken oder Gelegen verstärkt werden können und diese dabei so ausgelegt werden können, dass absolut gesehen die Querfestigkeiten mehr als die Längsfestigkeiten erhöht werden können. Beispiele für Vliesverbundstoffe sind in "Vliesstoffe: Rohstoffe, Herstellung, Anwendung, Eigenschaften, Prüfung" Herausgeber: W. Albrecht, H. Fuchs, W. Kittelmann (Wiley-VHH, Weinheim, New York, 2000), Kapitel 6.6.1 (Vliesverbundstoffe), S. 389-396 aufgeführt.

Die Herstellung bekannter Verbundstoffe ist aufwendig und teuer, insbesondere dann, wenn zusätzlich zu der den Vliesstoff verstärkende Schicht Klebstoffe oder Haftmassen benötigt werden. Aber selbst wenn zwei Faserflorlagen autogen, d.h. ohne weiteres Haftmittel, durch die verstärkende Schicht miteinander, verschweißt werden, so treibt dennoch die verstärkende Lage den Preis des Verbundstoffes übermaßen in die Höhe.

Im Vergleich zu Netzen, Geweben, Gewirken oder Gelegen sind monolithische, d.h. nicht-mikroporöse Folien, insbesondere wenn sie aus polyolefinischen Thermoplasten bestehen, besonders preisgünstig. Verbundstoffe aus Vliesstoffen und Folien sind ebenfalls bekannt. Solche Verbundstoffe können aber - je nach dem ins Auge gefassten Anwendungsgebiet - zahlreiche Nachteile haben. Die Folien neigen zum Rascheln (Geräuschbildung bei Bewegung bzw. beim Gebrauch), verringern die Textilität erheblich bezogen auf den Vliesstoff allein und verhindern eine Durchlässigkeit für die unterschiedlichsten Medien wie Luft, Gase und Flüssigkeiten, wie Wasser oder organische Lösungsmittel bzw. Dämpfe, wie z.B. Wasserdampf.

Der Einsatz einer undurchlässigen (und preisgünstigen) Folie in einem Laminat führt bekanntermaßen in einem Vliesstoff-Laminat zwar zu einer Festigkeitsverstärkung, hat aber den Nachteil, dass die Haptik bzw. die textilen Eigenschaften deutlich verschlechtert werden, die Steifigkeit erhöht wird und der Durchgang für Luft, Wasserdampf und gegebenenfalls anderer Gase extern stark - meistens bis auf Null -_herabgesetzt wird. Dies gilt insbesondere dann, wenn eine monolithische (d.h. porenfreie) Folie zur Laminierung verwendet wird. Die Versteifung des Laminates durch die Folie ist insbesonders dann stark ausgeprägt, wenn die Folie aus hartem Polymer, wie beispielsweise Polypropylen, aufgebaut ist und die Folie ganzflächig an die sie umschließenden Flächengebilde gebunden ist.

Eine Folienlaminierung auf Vliesstoff ist also nur dann angebracht, wenn eine Barriere-Wirkung erwünscht ist. Es ist also mit Verbundstoffen aus Vliesstoff und monolithischer Folie nicht möglich, durchlässige wie z.B. atmungsaktive Strukturen zu erzeugen. Eine deutliche Erhöhung der Querfestigkeiten ist damit zwar erreichbar. Wenn allerdings die kostengünstigste und meistens praktizierte Extrusionsbeschichtung (auch Cast Extrusion) direkt aus einer Breitschlitzdüse erfolgt, sind die Festigkeitszuwächse relativ bescheiden, weil bekanntermaßen erst eine Reckung zu hohen Folienfestigkeiten führt. Eine gereckte Folie kann aber wiederum nicht direkt aus der Düse sondern in einem zweiten Folgeschritt (nach der Reckung) auf dem Vliesstoff appliziert werden und in den meisten Fällen wird die Anwendung eines zusätzlichen Klebers, wie z.B. Hotmelt, notwendig. Dieses verkompliziert die Verbundstoff-Herstellung zusätzlich, macht sie teuer und führt dennoch nur zu einem völlig undurchlässigen Produkt.

### Darstellung der Erfindung

Aufgabe der vorliegenden Erfindung ist es, in einem Verbundstoff mit hauptsächlichem Vliesstoffanteil die Festigkeiten zumindest in Richtung quer zur Maschinenlaufrichtung um ein Mehrfaches zu erhöhen unter Beibehaltung einer hohen Durchlässigkeit des entstandenen Verbundes.

Gelöst wird diese Aufgabe durch den Verbundstoffe sowie dessen Herstellung gemäß den Patentansprüchen.

Die Erfindung betrifft einen Verbundstoff umfassend mindestens einen Vliesstoff und ein damit thermisch verbundenes, in Form von regelmäßig wiederkehrenden Mustern angeordnetes Verstärkungsmaterial, das sich von einer Folie aus thermo-plastischem Material ableitet.

Die Zahl der Schichten des erfindungsgemäßen Verbundes kann beliebig hoch sein, beispielsweise zwei bis sechs Schichten.

Bevorzugt wird ein Verbundstoff, der mindestens drei Schichten in der Abfolge Vliesstoff / Verstärkungsmaterial / Vliesstoff aufweist.

Der erfindungsgemäß eingesetzte Flor, der den Vliesstoff ausbildet, kann ein Spunbond sein oder bevorzugt ein Stapelfaservliesstoff. Der erfindungsgemäß eingesetzte Flor besteht bevorzugt aus in Maschinenlaufrichtung orientierten Stapelfasern.

Das Material, aus dem der Vliesstoff besteht, kann beliebig sein. Beispiele dafür sind Fasern aus natürlich vorkommenden Materialien, wie Baumwolle, oder halbsynthetisch hergestellte Fasern, wie Cellulosefasern, oder insbesondere Kunstfasern aus synthetischen Polymeren, vorzugsweise aus Polyester oder Polyamid.

Das thermoplastische Material, aus dem die Folie besteht, kann ebenfalls beliebig sein. Beispiele dafür sind thermoplastische Polymere, wie Polyamid, Polyester oder insbesondere Polyolefine. Dabei kann es sich um Homo- oder Copolymere handeln, vorzugsweise um Polyethylen oder ganz besonders bevorzugt um Polypropylen.

Üblicherweise ist die zum Einsatz kommende Folie eine uni- oder biaxial gereckte Folie.

In einer weiteren bevorzugten Ausführungsform ist der Vliesstoff mit der Folie durch thermisches Verschweißen mit mindestens einer Oberfläche des Vliesstoffs in der Form von regelmäßig wiederkehrenden Mustern verbunden, und die Folie ist an diesen Stellen der Oberfläche des Vliesstoffs erhalten geblieben und liegt an anderen Stellen der Oberfläche des Vliesstoffs infolge Zerreißens der Folie nicht mehr vor.

Bei dem regelmäßig wiederkehrenden Muster der thermisch verschweißten Stellen von Vliesstoff und Folie handelt es sich vorzugsweise um regelmäßig senkrecht zur und/oder in Maschinenlaufrichtung angeordneten Linien oder Balken.

In einer weiteren bevorzugten Ausführungsform des regelmäßig wiederkehrenden Musters der thermisch verschweißten Stellen von Vliesstoff und Folie liegt dieses in der Form von regelmäßig angeordneten Linien in der Form von Hexagons vor.

Der erfindungsgemäße Verbundstoff kann beliebige Flächengewichte aufweisen, beispielsweise im Bereich von 15 bis 250 g/m². Besonders bevorzugt sind Typen mit Flächengewichten von 20 bis 150 g/m².

Bedingt durch das nachstehend beschriebene Herstellungsverfahren kann der erfindungsgemäße Verbundstoff eine geringe dreidimensionale Struktur aufweisen, d.h. man findet in Bezug auf die Flächenebene alternierend auftretende Erhebungen und Vertiefungen geringer Höhe bzw. Tiefe.

Vorzugsweise ist der erfindungsgemäße Verbund im wesentlichen flächig und weist im wesentlichen keine in Bezug auf die Flächenebene alternierend auftretende Erhebungen und Vertiefungen auf.

Der erfindungsgemäße Verbundstoff lässt sich durch ein neues Verfahren herstellen, bei dem ausgehend von einer monolithischen, biaxial- oder zumindest monoaxial quer zur Maschinenlaufrichtung gereckten Folie in der Mitte oder mindestens einer Oberfläche des Verbundstoffes diese Folie während und/oder nach dem Verbinden mit dem Vliesstoff in der Form eines regelmäßig angeordneten Musters, vorzugsweise mittels Kalander-Verfestigung mit speziellen Gravuren, zu einem geöffneten Flächengebilde mit Perforationen und kontinuierlich miteinander verbunden Folienbestandteilen umgeformt wird, wie z.B. zu Folienbändchen oder Foliennetzstrukturen.

Die Erfindung betrifft daher auch ein Verfahren zur Herstellung des oben beschriebenen Verbundstoffs umfassend die Maßnahmen:
a) Kombination von mindestens einem Flor mit einer schrumpffähigen Folie aus thermoplastischem Material,
b) thermisches Verschweißen zwischen dem Flor und der schrumpffähigen Folie in Form eines regelmäßig wiederkehrenden Musters,
c) Erhitzen des erhaltenen Verbundes während oder nach Schritt b) auf eine solche Temperatur, dass sich der Vliesstoff ausbildet, der Schrumpf der schrumpffähigen Folie ausgelöst wird und sich regelmäßig in Bezug auf die Flächenebene alternierend auftretende Erhebungen und Vertiefungen ausbilden,
d) Verstrecken des Verbundes zumindest in einer Richtung, so dass die Erhebungen und Vertiefungen im wesentlichen verschwinden und die Folie an den nicht mit dem Vliesstoff verschweißten Stellen reißt.

Das Verfahren beruht auf einer Schwächung der gereckten Folie an den Rändern der Verschweißstellen, einer Schrumpfung der Folie, einer Nichtschrumpfung der Vliesstofflagen unter temporärer Ausrichtung von Erhebungen des Vliesstoffes in die dritte Dimension und Wiederherausziehen des Schrumpfes unter Makro-Öffnung der vorzugsweise mittellagigen Folie an den geschwächten Stellen zu einem durchlässigen Gebilde.

Das Wiederherausziehen des Schrumpfes kann vollständig oder nur unvollständig erfolgen.

Mit einem neuen Kompositaufbau und dem Verfahren zur Herstellung desselben können die oben genannten Nachteile behoben und die mechanische Festigkeit in mindestens einer gewünschten Richtung, beispielsweise quer zur Maschinenlaufrichtung, um ein Vielfaches angehoben werden.

Die Erfindung ermöglicht auf eine sehr einfach praktizierbare Weise aus den beiden Ausgangsmaterialien Fasern bzw. bereits vorgebildeten faserigen, porösen und wasserdampf- und luftdurchlässigen Flächengebilden und einer undurchlässigen Folie ein hochatmungsaktives mehrlagiges, beispielsweise dreilagiges Komposit zu schaffen mit sehr hohen mechanischen Festigkeiten sowohl in Maschinenlaufrichtung als auch quer zu dieser Maschinenlaufrichtung.

Mit der Erfindung ist es gelungen trotz des Einsatzes einer undurchlässigen Folie ein hochatmungsaktives und textiles Komposit zu bilden, das von den Vorteilen der Festigkeitsverstärkung durch die Folie profitiert, aber die Nachteile der Textilitätsverschlechterung und Undurchlässigkeit durch die Folie nicht aufweist, selbst wenn die Folie nicht mikroporös ausgebildet ist, eine monolithische Struktur aufweist.

In einer bevorzugten Ausführungsform besteht das dreilagige faserige Komposit aus einer Faserlage (1) mit einer Oberfläche (1a), einer zweiten Faserlage (2) mit einer der Oberfläche (1a) gegenüberliegenden Oberfläche (2a). Zwischen den Faserlagen (1) und (2) sind kontinuierliche Folienstreifen (3) mit vorgegebenem Muster eingelagert, wobei dieselben - quasi in situ - erst in dem Komposit während der Kompositherstellung entsprechend dem erfindungsgemäßen Verfahren aus einer Folie erzeugt worden ist.

Unter kontinuierlichen Folienstreifen oder Folienbändchen werden im Rahmen der vorliegenden Beschreibung entweder parallel zueinander in einer Vorzugsrichtung orientierte Bändchen mit jeweils gleichem oder sich abwechselnd wiederholenden unterschiedlichen Abständen oder kontinuierliche Muster aus Folienstreifen verstanden, wobei an den Überkreuzungen oder Überschneidungen der Folienstreifen keine Polymermasseanhäufungen auftreten, im Gegensatz zu beispielsweise den bekannten extrudierten Netzen oder Gelegen.

Die kontinuierlich Folienbändchenstrukturen können beliebige Formen oder Muster aufweisen. Stellvertretend seien genannt parallel zueinander ausgerichtete lineare oder gekrümmte Folienbändchen, zickzackförmige Bändchen genau parallel zueinander oder spiegelbildlich zueinander ausgerichtet. Auch netzartige Strukturen mit drei-, viereckigen oder vieleckigen Hohlstellen zwischen den Stegen, mit z.B. hexagonaler Anordnung sind denkbar. Im letzteren Fall wird auch von sog. Honigwabenstrukturen (Honeycomb) gesprochen, die sich insbesondere auch in der Natur und nachgeahmt in vielen technischen Anwendungen wiederfinden. Unter Bändchen werden im Rahmen dieser Beschreibung Strukturen mit nicht rundem sondern rechteckigem Querschnitt verstanden.

### Kurzbeschreibung der Zeichnung

Die nachfolgenden Abbildungen erläutern die Erfindung näher. Ferner werden bevorzugte Ausführungsformen des erfindungsgemäßen Verbundstoffes beschrieben.
- Figur 1: beschreibt eine Form des erfindungsgemäßen Verbundstoffes in Aufsicht.
- In Figur 2: ist der Querschnitt des Verbundstoffes aus Abbildung 1 entlang der Linie A-A schematisch dargestellt.
- Figur 3: beschreibt eine Fertigungslinie zur Herstellung des erfindungsgemäßen Verbundstoffes.
- Figuren 4a bis 4j: zeigen verschiedene Muster der Folienöffnungen im erfindungsgemäßen Verbundstoff.
- Figuren 5a bis 5c: beschreiben Oberflächenformen der Gravuren.
- Figur 6: beschreibt die einzelnen Stufen der Bildung von regelmäßigen Folienöffnungen im erfindungsgemäßen Verbundstoff

### Ausführung der Erfindung

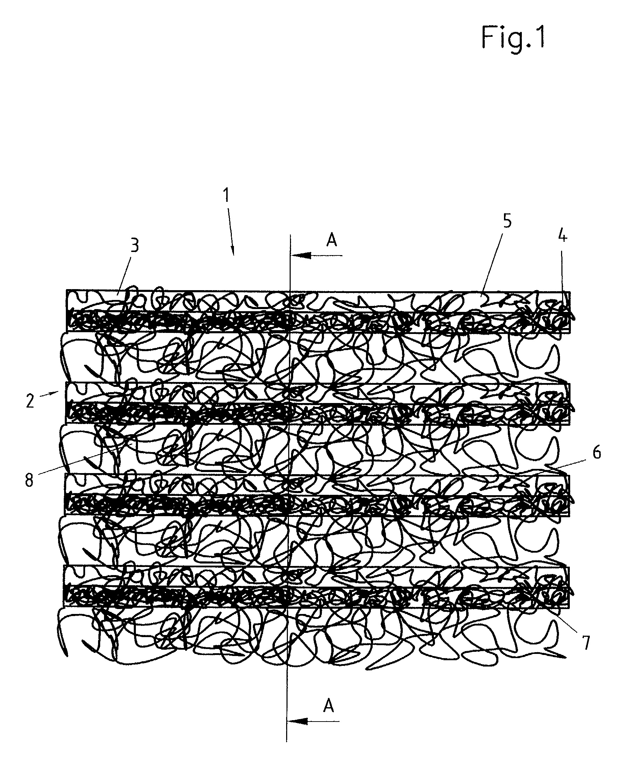

In Abbildung 1 ist ein Beispiel eines erfindungsgemäßen Verbundstoffes (1) in der Aufsicht widergegeben. Dieser besteht aus parallel zueinander angeordneten Folienstreifen (2). Innerhalb der Folienstreifen (2) lassen sich zwei unterschiedliche Zonen (3) und (4) erkennen. Die Fasern (5) des Vliesstoffes sind an den Folienzonen (4) mit der Folie innig verschweißt, während in der zur Zone (4) benachbarten Zone (3) die Fasern mit der Folie nicht verschweißt sind. Das Komposit (1) ist auch dadurch besonders gekennzeichnet, dass die Flächenbereiche (8) zwischen den Folienbändchen (2) hochdurchlässig gegen Gas und flüssige Medien sind. Die Flächenbereiche (8) bestehen ausschließlich aus Fasern.

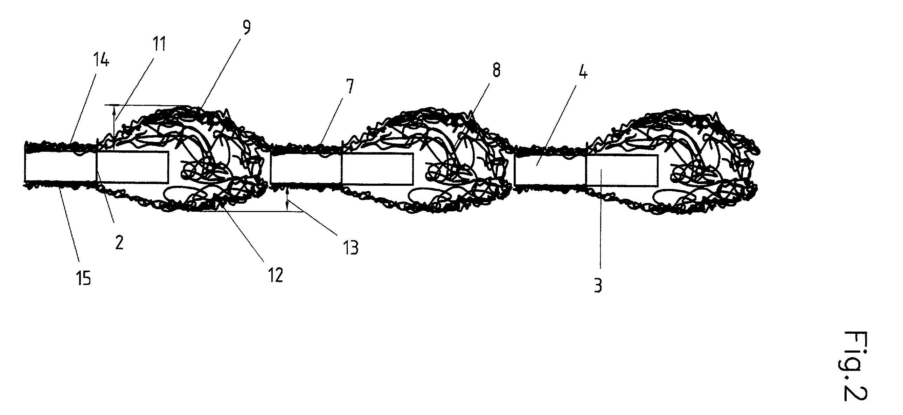

In Abbildung 2 ist der Querschnitt des Komposites entlang der Linie A-A schematisch dargestellt. Die Linie A-A kann der Maschinenlaufrichtung, der Richtung senkrecht zur Maschinenlaufrichtung oder irgend einem Winkel zur Maschinenlaufrichtung entsprechen.

Das Flächengebilde (1) weist also sich rhythmisch wiederholende, unterschiedliche drei Bereiche auf. Die Folienstreifenbereiche (4), die mit den Fasern verschweißt sind, die Folienstreifenbereiche (3), die mit den Fasern nicht verschweißt sind und die Bereiche (8), die in diesem Bereich ausschließlich aus ungebundenen Fasern bestehen. Durch die innige Verschweißung der Fasern (5) mit den Folienbereichen (4) entstehen einerseits in beiden Richtungen sehr hohe Festigkeiten und zum anderen ein hoher Faserbausch in den Bereichen (8). Durch diese Konstruktion können also an sich einander völlig wiedersprechende Eigenschaften in dem Laminat miteinander verbunden werden.

Die Folienstreifen (2) sind in Abbildung 2 im Querschnitt dargestellt. Die beiden Seiten B und C der Folienstreifen (2) sind in der dargestellten Ausführungsform in den Bereichen (4) oben mit gröberen Fasern (5), die ein sehr gutes Wiedererholvermögen aufweisen, und unten mit relativ feinen Fasern (10) eines niedrigen Wiedererholvermögens verschweißt. Der aus den Fasern (10) bestehende Faserflor des Flächengebildes (1) ist vorzugsweise bei der Kalandrierung einer glatten Walze und der aus den groben Fasern aufgebaute Faserflor einer gravierten Walze zugewandt. Aufgrund dieser Rahmenbedingungen bildet sich Komposit mit völlig unterschiedlicher Bauschhöhe ober- und unterhalb der Folienstreifen (2). Unter der Bauschhöhe (11) bzw. (13) wird im Rahmen der vorliegenden Beschreibung der Abstand zwischen Folienstreifenoberkante und der Stelle (9) mit der höchsten Erhebung auf der Oberseite B bzw. der Folienstreifenunterkante und der Stelle (12) mit der höchsten Erhebung auf der Unterseite C verstanden.

Die beiden Bauschhöhen können insbesondere durch die Wahl der chemischen Faserzusammensetzung, des Fasertiters, der Form des Faserquerschnittes, des Fasergewichts, der Gravurtiefe, des Gravurmusters, der Art der Kräuselung (zweidimensional oder spiralförmig = helikal) beeinflusst werden. Die Wahl dieser Parameter auf der Seite B und der Seite C kann der jeweiligen Anwendung bzw. deren Anforderungen angepasst werden. Hohlfasern und spiralgekräuselte Grobfasern werden vorzugsweise dann eingesetzt, wenn ein besonders hohes spezifisches Volumen, d.h. ein hoher Bausch und eine gutes Wiedererholvermögen nach Druckbelastung in der Kälte oder der Wärme verlangt wird.

In Abbildung 2 ist der Fall eines hohen Bauschvermögens auf der Seite B und eines sehr niedrigen auf der Seite C dargestellt. An den Folienstreifenbereichen (4) sind sowohl die Grobfasern der Seite B an der Folienstreifenoberfläche (14) als auch die Feinfasern (10) an der anderen Folienstreifenoberfläche (15) innig mit der Folie verschweißt. Ein hoher Bausch gepaart mit hoher Festigkeit der bauschigen Bereiche (8) an den Verschweißstegen (4) erleichtert beispielsweise das Einhaken des Einhakungsteiles von mechanischen Verschlusssystemen und ergibt hohe Schälund Scherfestigkeiten. Feinfasern, wie sie beispielsweise auf der Seite C der Abbildung 2 eingesetzt worden sind, ergeben relativ glatte, weiche, nicht hautreibende Oberflächen, so dass solche Oberflächen in Kontakt mit dem menschlichen Oberkörper ohne Gefahr der Auslösung von Hautirritationen kommen können.

Mit der Erfindung ist man also in der Lage, einander völlig widersprechende Eigenschaften in idealer Weise einem Komposit einzuverleiben.

Die erfindungsgemäß zum Einsatz kommende Folie muss mindestens in einer Vorzugsrichtung gereckt sein. Das Verhältnis der Länge vor zu nach der Reckung wird als Reckverhältnis bezeichnet. Hat die Folie beispielsweise nach der Reckung eine Längung auf das 4-fache ihrer Ursprungslänge erfahren, so wird von einem Reckverhältnis von 5/1 gesprochen. Zur Erreichung einer möglichst hohen Schrumpfkraft sollte die Folie stark gereckt sein und ein Reckverhältnis von mindestens 2/1 aufweisen. Die obere Grenze der Reckung ist durch seine Höchstzugkraft in der Reckrichtung begrenzt.

Für die Erfindung ist es besonders vorteilhaft, wenn die Reckung der Folie in zwei Vorzugsrichtung erfolgt ist, d.h. in Maschinenlaufrichtung und quer zu ihr. Ein hoher Reckgrad sowohl in Maschinenlaufrichtung als auch quer zur Maschinenlaufrichtung beeinflusst das Ergebnis der Erfindung besonders günstig, da dann die Festigkeit des Komposites nach dem erfindungsgemäßen Verfahren quer zur Maschinenlaufrichtung besonders stark erhöht wird, beruhend auf der Lehre, dass Reckungen und deren damit verbundene Kristallinitätserhöhung die mechanischen Festigkeiten deutlich verbessert. Sofern auf den besonders hohen Festigkeitszuwachs verzichtet werden kann, ist auch der Einsatz einer nur unidirektional gereckten Folie denkbar.

Als Polymerrohstoff für die erfindungsgemäß eingesetzte Folie kann im Prinzip jeder extrudierbare und zur Folie verformbare thermoplastische Kunststoff verwendet werden. Solche Thermoplaste, die zu besonders starker Kristallisation nach Reckung neigen sind bevorzugt. Die Natur des Thermoplasten der gereckten Folie muss zum Zwecke einer intensiven Verschmelzung an den autogenen Verschweißstellen mit zumindest einem Teil der Fasern der mit dieser zu verbindenden Vliesstofflage, vorzugsweise der beiden sie umgebenden Vliesstofflagen, vorzugsweise gleich oder ähnlich sein. Wird beispielsweise eine biaxial gereckte Schrumpffolie aus Polypropylen ("PP-Schrumpffolie") verwendet, so sollte zumindest ein Faseranteil der Vliesstofflagen ebenfalls aus Polypropylen ("PP") bestehen.

Die Folie kann nach den bekannten Reckverfahren gereckt worden sein. Das Blasformen durch eine Runddüse führt zu einer biaxialen Reckung. Die Folienextrusion kann auch durch eine Schlitzdüse (Chill Roll Verfahren) erfolgen, der nach bekannten Methoden eine Reckung in Maschinen- und quer zur Maschinenlaufrichtung oder nur eine Reckung in einer der beiden Vorzugsrichtungen angeschlossen wird.

In dem Polymer der Folie können Zusatzstoffe, wie Farb- oder Weißpigmente, antimikrobielle Wirkstoffe, Antistatika, Stabilisatoren oder Hydrophilierungsmittel eingeschlossen oder eingelagert (eingeschmolzen) sein. Diese Agenzien seien nur stellvertretend für viele weitere andere genannt. Diese Folienzusatzstoffe können in einem immobilisierten oder auch mobilisierten Zustandform in der Folie vorliegen, d.h. sie können dort bei Lagerung mit oder ohne Temperaturbeaufschlagung an ihrem Eintragungsort verbleiben oder an die Oberflächen der Folie migrieren, um dort ihre ihr zugewiesene Wirkung auszuüben. Die Folie kann auch vor oder nach der Reckung durch Applikation von außen veredelt worden sein. Stellvertretend für solche Veredelungen seien genannt eine Behandlung durch Niederdruck- oder Atmosphärenplasma, Metallbedampfung im Hochvakuum oder Beschichtungen.

Als Folienextrudat können auch Copolymere oder Blends (Verschnitte) eingesetzt werden.

Für die Herstellung eines Komposites aus verstreckten PP-Stapelfasern und PP-Streifen in der Mittellage ist es zum Zwecke der optimalen Verbundfestigkeit vorteilhaft, wenn eine PP-Folie als Ausgangsmaterial eingesetzt, die nach dem Chill Roll Verfahren hergestellt worden ist und maximal möglich in Maschinenlaufrichtung und quer zur Maschinenlaufrichtung gereckt worden ist. In diesem Fall handelt es sich um eine PP-Folie, die in Maschinenlaufrichtung im Verhältnis 1:5 und quer zur Maschinenlaufrichtung 1:9 bis 1:10 gereckt worden ist. Solche Folien sind hochtransparent und weisen einen Schmelzpunkt von ca. 150 bis 155°C auf und damit gleich dem Schmelzpunkt von PP-Stapelfasern. Die Höchstzugkräfte solcher PP-Folien sind quer zur Maschinenlaufrichtung in etwa doppelt so hoch als in Maschinenlaufrichtung. Auch zum Zwecke der Erreichung besonders hoher Querfestigkeitssteigerungen sind solche Folien ganz besonders geeignet. Werden als Faserschichten PP-Spinnvliesstofflagen eingesetzt, so kann es zum Zwecke der optimalen Verbundfestigkeit vorteilhaft sein, in Maschinenlaufrichtung nur eine Teilverstreckung und quer zur Maschinenlaufrichtung eine Teil- bis Vollverstreckung durchzuführen.

Die Folie ist vorzugsweise eine gereckte monolithische Folie. Sie kann jedoch auch aus einer quer zur Maschinenlaufrichtung monoaxial oder biaxial gereckten Folie mit Mineralstoff-Füllung, wie beispielsweise mit einem adhäsiven Agenz überzogene Kreide zum Zwecke der Erzeugung einer Mikroporösstruktur bestehen, obschon eine solche Folie für das Verfahren nicht besonders vorteilhaft ist, da die Folie durch die Mikroporosität geschwächt ist und Durchlässigkeit von Wasserdampf durch die geschaffenen Makroporen im fertigen Verbundstoff geschaffen werden.

Die beiden Faserschichten als Ausgangsmaterial des in Abbildungen 1 und 2 dargestellten erfindungsgemäßen 3-lagigen Vlies / Folienbändchen / Vlies-Kompositaufbau können als lose, völlig ungebundene mit trockenen Vliesablegemethoden gebildete Faserflore vorliegen, zwischen die vor der Kalanderverfestigung/Lamininierung als drittes Ausgangsmaterial die Folie eingeführt wird. Die Fasern können als geschnittene Stapelfasern oder als Endlosfilamente vorliegen. Sie können 2-dimensional und/oder 3-dimensional (= helical = spiralförmig) gekräuselt sein. Auch Verschnitte von gekräuselten mit- ungekräuselten Fasern sind denkbar und ebenso Vermengungen von Endlosfasern mit Kurzschnitt- oder Stapelfasern (sog. Coform-Produkte). Die beiden Faserlagen sind gewöhnlich entweder mechanisch oder aerodynamisch gereckt oder verstreckt worden. Es ist jedoch auch denkbar, den verstreckten Fasern auch solche entweder des gleichen oder unterschiedlichen Polymeraufbaues zuzumischen, die nur teilweise (partiell) oder überhaupt nicht verstreckt worden sind.

Zur Faserablage auf ein Transportband oder Stützmedium eignen sich alle bekannten Vlieslegemethoden. Im Falle einer Vlieslegung nach dem Fourdrinier-Prinzip (Nassvlies) muss allerdings vor der Kalandrierung zu dem Komposit das Wasser durch Trocknung zuvor entfernt werden. Sofern das Wasser allerdings einen weichmachenden Effekt auf die Faser oder zumindest eine der Faserkomponenten im Falle eines Verschnittes ausübt, kann eine geringe Restfeuchte im Faserflor verbleiben.

Eine der beiden oder beide Faserlagen können vor der Kalandrierung unter Hitze und Druck bereits durch bekannte Methoden der Vliesstoffverfestigung vorgebunden sein, wobei die Bindung partiellflächig oder ganzflächig sein kann.

Der Einfachheit und der Kosten wegen wird jedoch zumindest bei Stapelfaserschichten vorzugsweise auf eine Vorbindung verzichtet.

Prinzipiell kann das gesamte Spektrum der Faserdurchmesser und Faserquerschnitte eingesetzt werden. Auch Fasertiterverschnitte sind möglich. Einschränkungen sind lediglich dadurch gegeben, dass die mit steigenden Titer zunehmende Fasersteifigkeit den Schrumpf zunehmend behindert. Sofern also in dem Verbundsstoff besonders hohe Flächenanteile ohne Folienstreifen oder -fragmente, d.h. besonders hohe Luft- und Fluiddurchlässigkeiten gewünscht werden und hohe Textilität erwünscht wird, sind sehr grobe Titer nicht vorteilhaft.

Die Wahl des Titers wird also von den o.g. Überlegungen und natürlich durch die Anforderungen der unterschiedlichsten Anwendung mitbestimmt.

Die Titer der in den Faserlagen verwendeten Fasern liegen im Bereich von ca. 0,05 bis ca. 50 dtex. Fasern unter ca. 0,5 dtex werden vorzugsweise durch mechanische oder hydrodynamische Splittung aus Bikomponenten-Fasern unterschiedlichsten Ouerschnittes erzeugt.

Dem Fachmann ist es geläufig, dass zum Zwecke einer guten Verbundfestigkeit zumindest Verschnittkomponenten der Fasermischung aus solchen Thermoplasten bestehen die chemisch gleich oder zumindest ähnlich aufgebaut sind und gleiche oder sehr ähnliche Erweichungs- bzw. Schmelzbereiche aufweisen. Die Anforderungen an die Verbundfestigkeit sind unterschiedlich in Abhängigkeit von der Anwendung des erfindungsgemäßen Kompositaufbaues.

Die Fasern können hydrophob aviviert, von Haus aus bereits hydrophob oder auch hydrophiliert oder vom Polymer aus bereits hydrophil ausgestaltet sein. Die Fasern können voll- oder halbsynthetisch sein. Das Wasseraufnahmevermögen und Rückhaltevermögen kann durch Zumischen von cellulosischen Fasern, wie Zellwolle, Lyocell, Baumwolle oder Zellstoff auf die von der Anwendung gewünschter Niveau gehoben werden. Die Fasermischung kann auch anteilig superabsorbierende Partikel in Faser-, Puder- oder Pulverform enthalten

Die Fasern des erfindungsgemäß eingesetzten Vliesstoffes können in Maschinenlaufrichtung, quer zur Maschinenlaufrichtung und in sog. Wirrlage, d.h. anisotropisch abgelegt worden sein. Die quer zur Maschinenlaufrichtung abgetäfelt abgelegten Flore können zum Zwecke der Geschwindigkeitssteigerung zusätzlich mit sogenannten Acceleratoren in Maschinenlaufrichtung bis zu verschiedenen Graden umorientiert worden sein. Die Wirr-Ablage der Fasern kann aerodynamisch erfolgt worden sein.

Die Faserorientierung wird weitgehend von den Anorderungen der mechanischen Festigkeit in Maschinenlaufrichtung (nachfolgend auch als "md" bezeichnet) und quer zu derselben (nachfolgend auch als "cd" bezeichnet) bestimmt. Bei dem erfindungsgemäßen Verfahren hat sich auch gezeigt, dass mit Faserorientierungen quer (d.h. im 90° Winkel) zur gewünschten Schrumpfrichtung die vergleichsweise höchsten Schrumpfwerte erzielt werden. Soll beispielsweise bei einem Verbund Faserlage/Vliesstreifen aus Folie/Faserlage, bei dem eine optimal gereckte PP-Schrumpffolie (1:5 in md und 1:9 in cd) als Ausgangsmittelschicht eingesetzt wurde, der Querschrumpf weitgehend unterbunden und der Längsschrumpf (in Maschinenlaufrichtung) dagegen gefördert werden, so ist eine Ablage des Faserflores quer zur Maschinenlaufrichtung vorteilhaft.

Die Erfindung zeichnet sich von allen anderen bekannten Bindungsmethoden dadurch aus, dass das Längs-Querverhältnis der Höchstzugkräfte im Verbundstoff gegenüber der reinen Vliesstoffkomponenten stark verändert werden kann.

Wird beispielsweise ein Vliesstoff, dessen Fasern in Maschinenlaufrichtung abgelegt worden sind, der anschließend durch eine bekannte Bindungsmethode verfestigt worden ist und eine Höchstzugkraft in Maschinenlaufrichtung von 50 N/5 cm Streifenbreite und 10 N/5cm quer zur Maschinenlaufrichtung aufweist, durch eine weitere Nachbehandlung, wie beispielsweise Bedrucken, Vollbadimprägnierung oder Schaumimprägnierung zusätzlich verfestigt, so wird die Höchstzugkraft zwar in beiden Vorzugsrichtungen erhöht, das Längs-/Querverhältnis der Höchstzugkräfte bleibt aber unverändert bei 5 : 1 (50 zu 10 N/5cm Streifenbreite).

Der erfindungsgemäße, atmungsaktive Verbundstoff zeichnet sich dagegen dadurch aus, dass durch das erfindungsgemäße Verfahren der Bildung einer durchlässigen Folienstruktur aus einer ursprünglichen monolithischen (undurchlässigen) Folie in Kombination mit dem Vliesstoff eine additive Festigkeitssteigerung entsprechend des absoluten Betrages der Folie ergibt. Ist eine PP-Folie beispielsweise durch das Blasverfahren gereckt worden, so weist sie ein gleiches Reckverhältnis in Maschinenlaufrichtung und quer zu derselben und somit auch gleiche Festigkeiten in diesem beiden Richtungen auf. Ist die Höchstzugkraft der PP-Folie beispielsweise 50 N/5cm in beiden Richtungen so weist das Komposit Höchstzugkräfte in Maschinenlaufrichtung von 50+50= 100 N/5cm und in Querrichtung 10+50 = 60 N/5cm auf. Das Verhältnis der Höchstzugkräfte längs/quer hat sich gegenüber dem des Vliesstoffes von 5/1 auf 10:6 = 1,67:1 also deutlich zugunsten der Querrichtung verändert.

Der Verbundstoff kann unterhalb und oberhalb der Folie bzw. der nach dem angewandten erfinderischen Methode Folienbändchen oder Foliennetz im Faserflächengewicht, der Faserzusammensetzung, dem Fasertiter bzw. Titergemisch gleich oder ungleich sein. Je nachdem entstehen dadurch im Aufbau symmetrische oder asymmetrische Strukturen. Darunter werden im Rahmen dieser Beschreibung spiegelbildlich zur Folie bzw. zum Folienbändchen oder Foliennetz gleiche oder ungleiche Strukturen verstanden. Aufgrund der Tatsache, dass gewöhnlich ein Kalanderwalzenpaar aus einer glatten und einer gravierten Walze besteht kann trotz gleichem Aufbau beidseitig der Folie eine optisch anmutende Asymmetrie erzeugt werden, mit höheren Erhebungen auf der Gravurwalzenseite und niedrigeren oder gar keinen Erhebungen auf der Glattwalzenseite.

Eine bevorzugte Variante des erfindungsgemäßen Verfahrens beruht darauf, dass in einem dreilagigen Komposit aus 2 Faserlagen außen und einer als Mittelschicht eingesetzten Folie, wobei die Folie entweder bereits nach Verlassen des Kalandernips oder nach anschließender zusätzlicher thermischer Beaufschlagung schrumpft unter Ausbildung von Wellungen oder anderweitigen Erhebungen senkrecht zur Folienebene. Dabei sind die Verschweißstellen an den Kanten des dreidimensionalen Komposites bereits derart geschwächt, dass durch Zugbe-anspruchung in Maschinenlaufrichtung, quer zu derselben oder in beiden Vorzugsrichtungen eine Längung des Flächengebildes unter Verschwinden der dreidimensionalen Struktur erfolgt. Durch die Streckung oder Reckung des Flächengebildes bis zum Verschwinden der Wellungen oder Erhebungen reist die Folie an den geschwächten Stellen unter Bildung von perforierten Strukturen oder Bändchen auf, wobei die Form der Perforationen oder Folienöffnungen durch die eingesetzte Gravur der Kalanderwalze weitgehend vorbestimmt wird.

Die Streckung oder Reckung kann unmittelbar nach Verlassen des Komposites mit der noch intakten, aber an den Konturen geschwächten Folie erfolgen oder in einem separaten Arbeitsgang. Die Reckung oder Streckung des Komposites in Maschinenlaufrichtung kann mit an sich bekannten Mitteln durch entsprechende Geschwindigkeitsdifferenzen in den Nips zweier Walzenpaare erfolgen, wobei zum Zwecke der Reckvergleichmäßigung und der Flächengewichtskonstanz über die gesamte Warenbreite zwischen dem Bremswalzenpaar und dem Reckwalzenpaar mit höherer Geschwindigkeit zahlreiche weitere, meist nicht eigenangetriebene Walzen angeordnet sind, die von dem zu reckenden Komposit umschlungen werden. In US-A-6,051,177 und US-A-5244,482 werden solche Reckmaschinen beschrieben. Die Reckung erfolgt vorzugsweise bei Temperaturen deutlich unter dem Erweichungspunkt der Folie bzw. unter dem Glasumwandlungspunkt der Folie und ganz besonders bevorzugt in der Kälte (bei Raumtemperatur).

Die Reckung quer zur Maschinenlaufrichtung erfolgt vorzugsweise entweder im Spannrahmen oder in ineinandergreifenden Rillenwalzen.

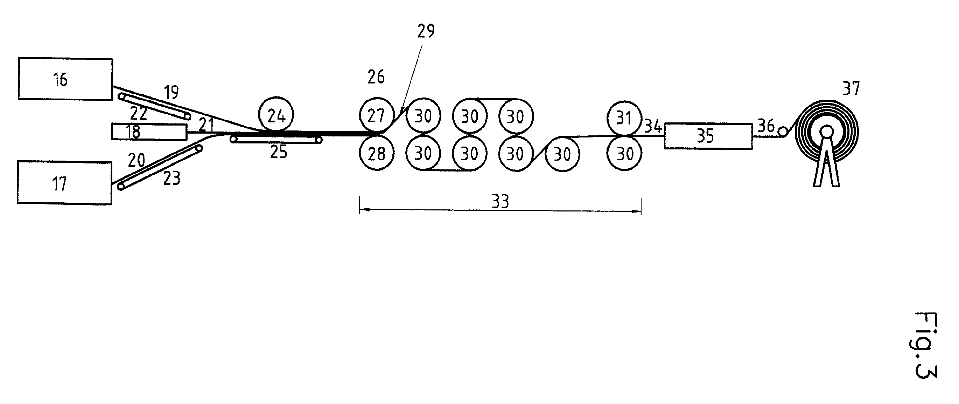

In Abbildung 3 ist eine Fertigungslinie zur Herstellung des erfindungsgemäßen Komposites schematisch dargestellt.

Aus Vliesbildungsvorrichtung (16) wird ein erster Faserflor (19) auf das obere Stützungsband (Lattenband) (22) abgelegt und aus der Vliesbildungsvorrichtung (17) ein zweiter Faserflor (20) auf das untere Stützungsband (23) abgelegt. Zwischen den ersten Faserflor (19) und den zweiten Faserflor (20) wird von der Seite her - d.h. im 90° Winkel zur Maschinenlaufrichtung - eine Folie (21) von der Folienzulaufvorrichtung (18) über ein 45° Schwert (in Abb. 3 nicht eingezeichnet) in Maschinenlaufrichtung umgelenkt, so dass die gereckte Folie (21) die Mittelschicht des aus erstem Faserflor (10), dem zweiten Faserflor (20) und der Folie gebildeten 3-lagigen Schichtmaterials bildet. Dieser 3-lagige Verbund wird von dem Stützungsband (25) in Maschinenlaufrichtung transportiert und zum Zwecke des besseren Transportes mit einer Andruckwalze (24) leicht zusammengepresst. Dies erleichtert auch die Einführung des 3-lagigen Schichtmaterials in den Pressspalt des aus den beiden Walzen (27) und (28) bestehenden Kalanders. Von den beiden Kalanderwalzen (27) und (28) ist eine der beiden mit einer Gravur versehen und der andere besitzt eine glatte oder leicht gerauhte Oberfläche. Das im Kalanderpressspalt verbundene 3-lagige Laminat wird über mehrere mit einer glatten Oberfläche versehene Walzen (30) geführt, wobei diese Walzen entweder selbst nicht angetrieben werden oder im Falle eines jeweiligen eigenen Antriebes die Drehgeschwindigkeit in Maschinenlaufrichtung von einer zur jeweils nächsten Walze immer mehr erhöht wird. Schließlich passiert die Ware (34) den Pressspalt des aus den beiden Zugwalzen (31) und (32) bestehenden selbst angetriebenen Presswerkes. Die Umlaufgeschwindigkeit der Walzen (31) und (32) bzw. die Transportgeschwindigkeit der Ware (34) im Pressspalt ist höher als diejenige des noch ungedehnten Verbundstoffes (29) beim Passieren des Walzenpaares (27) und (28). Das Verhältnis der Umlaufgeschwindigkeit der Walzen (31) und (32) zu (27) und (28) bestimmt das Reck- oder Dehnungsverhältnis. Die Reckung oder Dehnung in der Längsreckmaschine (33) wird in der Weise oder bis zu dem Ausmaß durchgeführt, dass damit im wesentlichen kein Breitenschwund (d.h. kein Neck-in) verbunden ist. Das Reckverhältnis ohne Breitenschwund wird bestimmt durch den Schrumpfbetrag, den die gereckte Folie (21) in Maschinenlaufrichtung bei der Kalanderpassage erleidet.

An den Ecken der Verschweißlinien tritt eine Schwächung auf, wenn die Kanten der Gravurlinien nicht abgerundet sind. Die Schwächung der Folie ist wesentliche Voraussetzung für das Funktionieren des Verfahrens. Bei Dehnungsbeanspruchung reißen die Verschweißstellen auf und ergeben nicht folienabgedeckte, atmungs-aktive Bereiche. Die Form der linienförmigen Öffnungen und damit die nach der Streckung verbliebenen mit Folienfragmentformen als Mittellage des Verbundstoffes sind also vorgegeben durch das Design der gravierten Walze und des Schrumpfbetrages der Folie. Durch die Dehnung in Laufrichtung werden beispielsweise die Wellungen der beiden Vliesstofflagen unter Aufplatzen an den geschwächten Rändern der Schweißstellen glattbezogen unter Verlust der dreidimensionalen Struktur.

Der Verbundstoff (34) kann nach der Reckung bereits aufgewickelt werden. Er kann aber auch, wie in Abbildung 3 dargestellt, zusätzlich noch in einem Spannrahmen mit Kluppen quergereckt werden, um auch Folienaufreißstellen in Querrichtung zu erzeugen. Durch die Querreckvorrichtung (35) erfährt der Verbundstoff (34) einen entsprechenden Flächenzuwachs und wird dabei in den breiteren Verbundstoff (36) überführt. Schließlich wird die Ware zu einer Masterrolle (32) aufgewickelt. Beim Einsatz von in Maschinenlaufrichtung monoaxial gereckter Folien, die deswegen ausschließlich in dieser Richtung schrumpfen, ist eine Querreckung (ohne Längeneinsprung) in der Regel weniger bevorzugt.

Außerdem ist festzustellen, dass das Verfahren den Einsatz von biaxial gereckten Folien auch insofern bevorzugt, als damit viel höhere Querfestigkeiten bzw. der Beitrag zur Querfestigkeitserhöhung um vielfaches höher ausfällt als mit einer quer zur Maschinenlaufrichtung nicht gereckten Folie.

Der Folien-Schrumpf in Maschinenlaufrichtung liegt üblicherweise im Bereich von 5 bis 60% und das Längsreckverhältnis dementsprechend im Bereich von 1 : 1,05 bis 1 : 2,50, nachdem sich dieses auf die geschrumpfte Ware bezieht.

Für Reckungen im Bereich von ca. 1 / 1,05 bis ca. 1 / 1,30 kann auf die Walzen (30) zwischen den Reckstellen (27) / (28) und (31) / (32) verzichtet werden. Für sehr hohe Reckverhältnisse von mehr als ca. 1 / 2,0 kann es ratsam sein, mit mehr als zwei Presswalzenspalten zu arbeiten. Im Prinzip sind solche Reckanlagen für die Herstellung von mono- und/oder biaxial gereckten Folien bekannt. Sie müssen nur auf die deutlich niedrigeren Reckverhältnisse für die erfindungsgemäßen Vliesstoff/Folie/Vliesstoff-Verbundstoffe angepasst werden.

In Abb. 3 ist der Fall einer In-line Fertigung bis zum erfindungsgemäßen Endmaterial dargestellt. Eine solche Linie bietet sich für die Erzeugung von offenen Folienflächen im Verbundstoff von ca. 5 % bis ca. 40 % an. Bei offenen Flächen von mehr als ca. 40% kann es ratsam sein, die Herstellung des Verbundes Faser/Folie/Faser mittels Hitze und Druck bis zur Stufe der noch nicht geschrumpften Folie in einem separaten Verfahrensschritt durchzuführen in einer zweiten Stufe zu schrumpfen und in einer dritten Stufe den Schrumpf bzw. die Wellung oder Erhebung unter Folienöffnung wieder herauszuziehen. Die Stufen zwei und drei können dabei wieder In-line gelegt werden.

Im Falle eines hohen Schrumpfes und der Erzeugung entsprechend großer offener Flächen und der Durchführung des Prozesses in zwei oder drei Stufen kann die erste Stufe der Verbundstoffherstellung mit noch intakter, ungeschrumpfter Folie anstelle mit Hitze und Druck auch durch Ultraschallverschweißung erfolgen. In einem separaten Arbeitsgang wird dann durch Hitzeeinwirkung geschrumpft und durch Anlegen einer Spannung in einer oder in beiden Vorzugsrichtungen die Folienöffnungen in der Mitte des Verbundstoffes erzeugt.

Das bevorzugte Verfahren ist jedoch die in Abb. 3 dargestellte In-line Methode.

Die Form der Folienöffnungen und der prozentuale Öffnungsgrad werden durch die Kalandergravur, den Schrumpfgrad und das Reckverhältnis in Maschinenlaufrichtung und/oder quer zur Maschinenlaufrichtung vorbestimmt. Voraussetzung für ein Gelingen der Erzeugung von Folienöffnungen ist eine gezielte Schädigung oder Schwächung des Komposites an zumindest einer der Außenkanten der Verschweißungsbereiche.

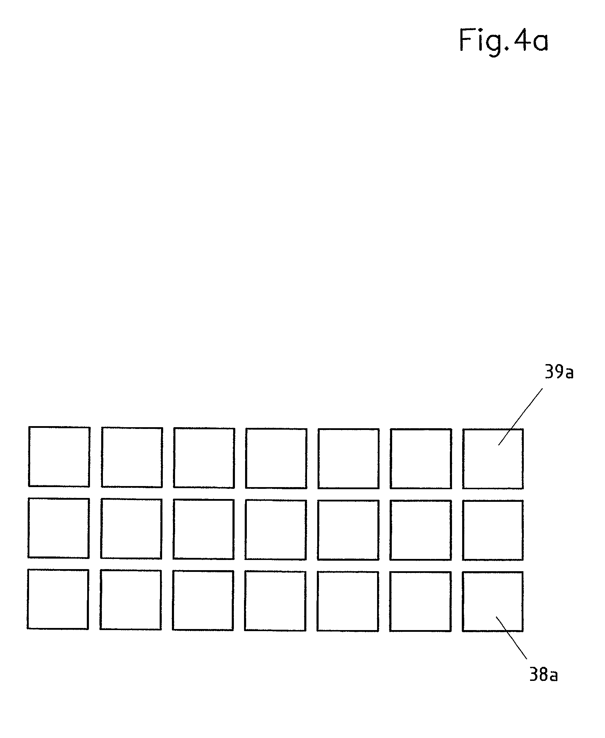

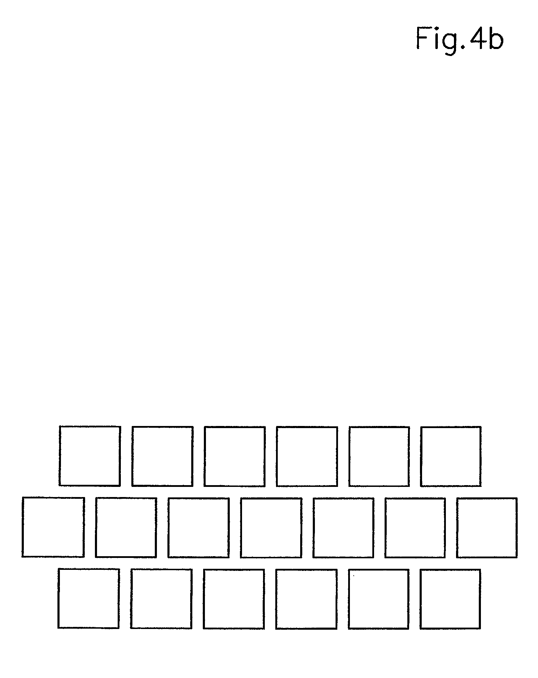

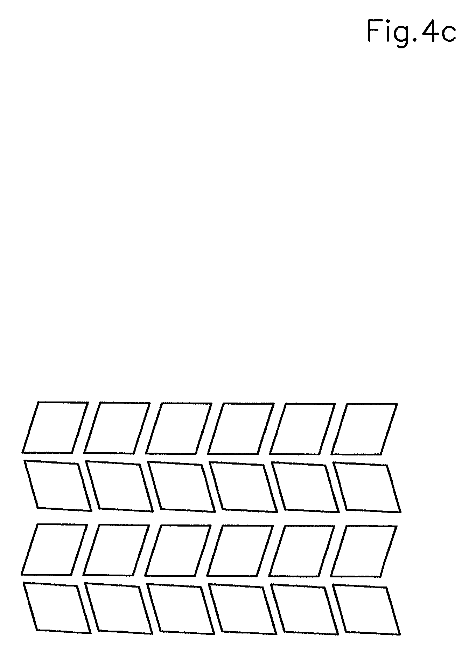

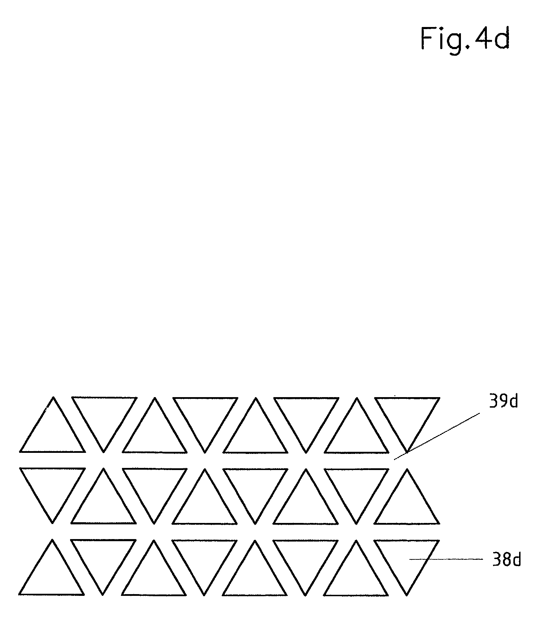

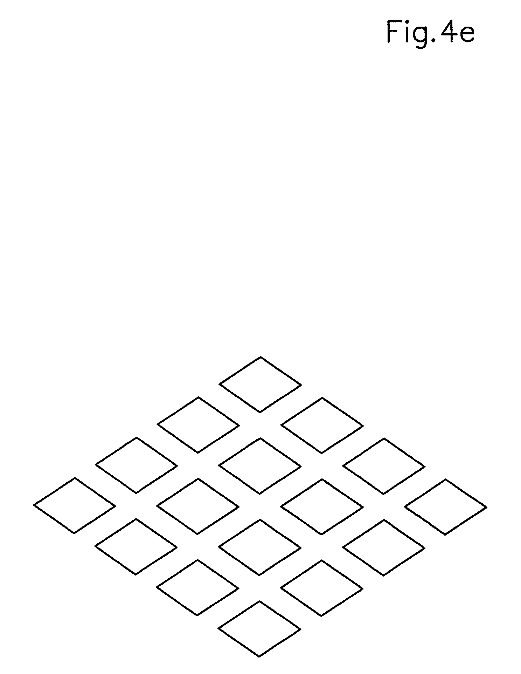

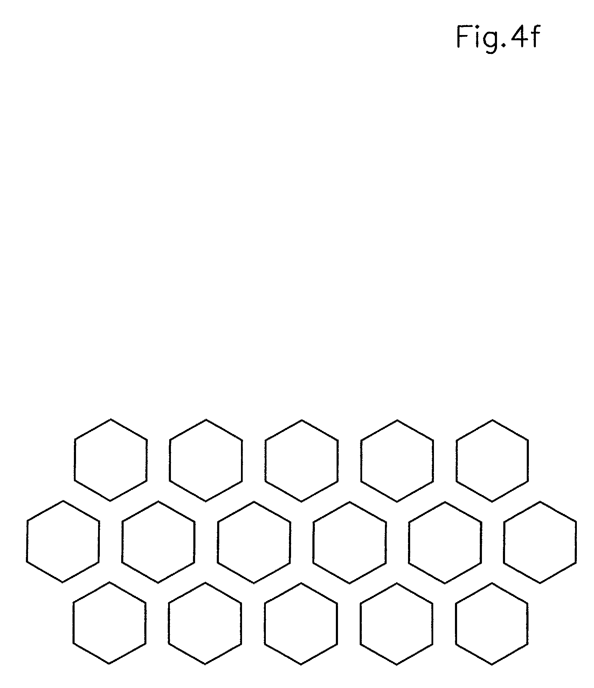

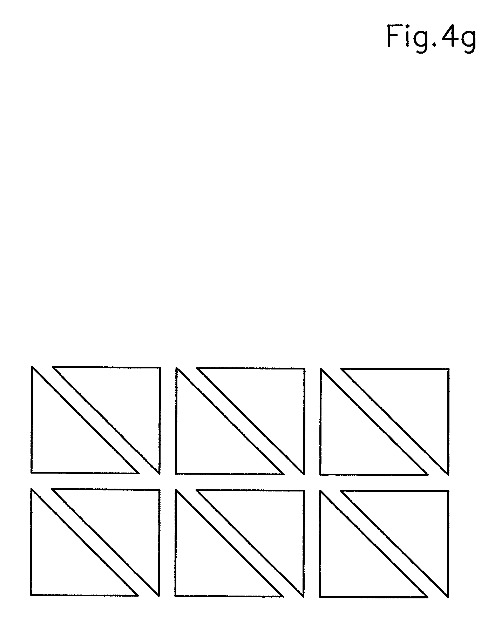

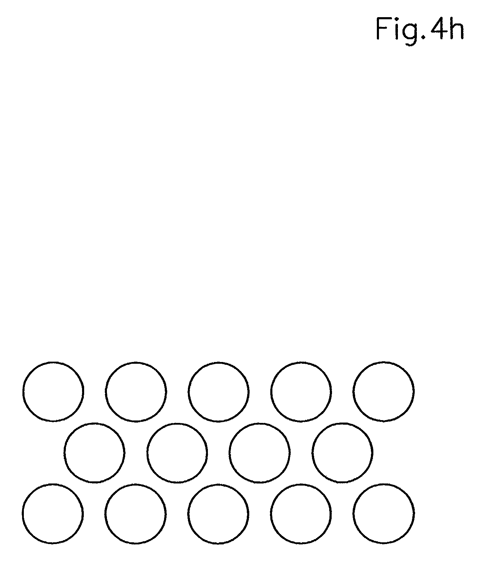

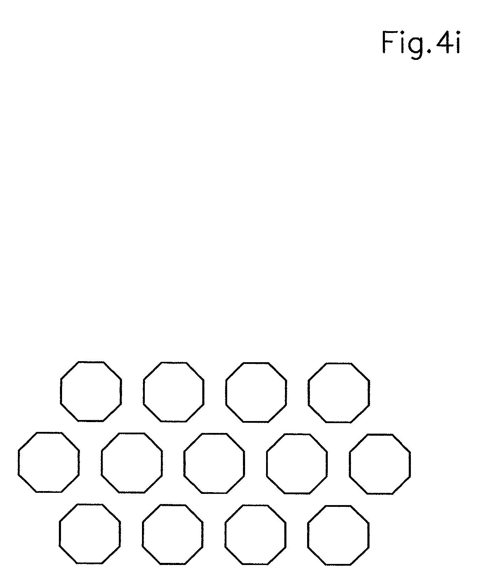

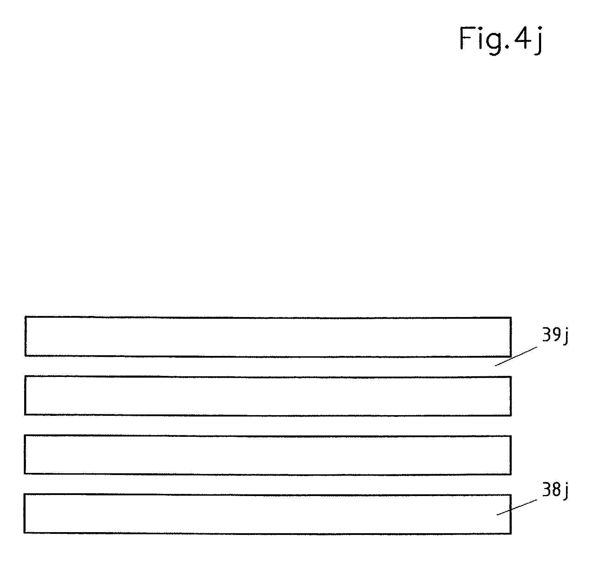

In den Abbildungen 4a bis 4j sind unterschiedliche Gravuren in der Draufsicht wiedergegeben:

Die erhobenen ununterbrochenen Bereiche der Gravurwalze sind in zwei Beispielen durch (39a), (39d) und (39j) gekennzeichnet und die Vertiefungen durch (38a), (38d) und (38j).

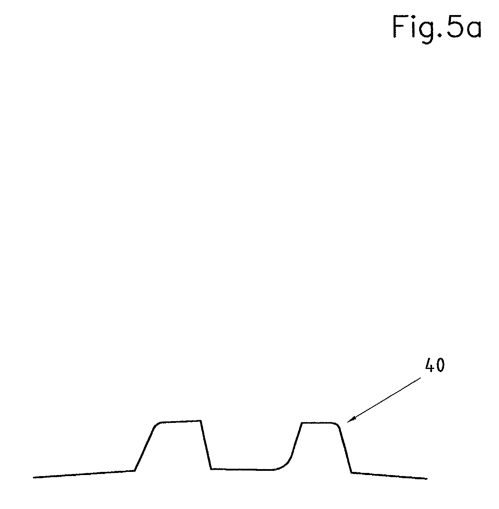

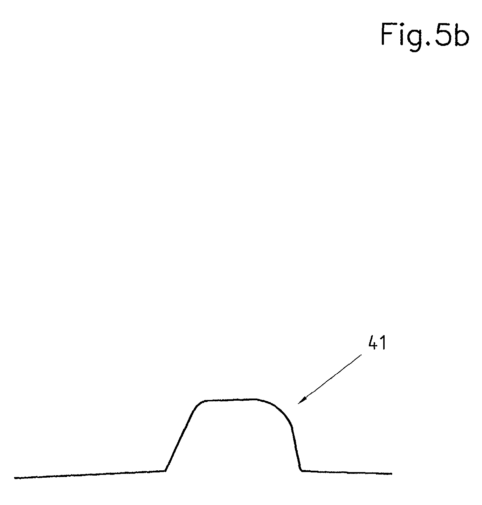

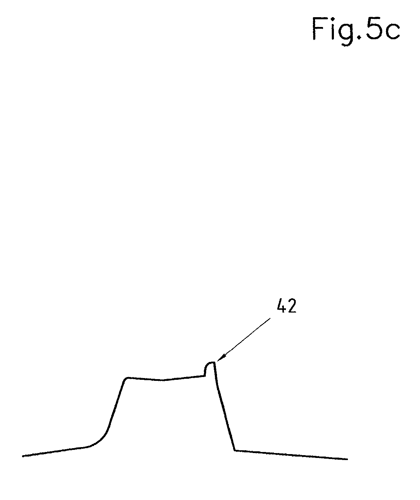

In Abbildung 5a ist ein Ausschnitt der Oberfläche einer gravierten Kalanderwalze im Querschnitt dargestellt. Die Kanten (40) der Gravurerhebungen sind scharfkantig ausgebildet. Dies ist wichtig für das erfindungsgemäße Verfahren, um eine Schwächung der Verschweißung an dieser Stelle zum Zwecke der späteren Öffnung der Folie nach dem Schrumpf zu erreichen. Ausschließlich abgerundete Ecken (41), wie in Abbildung 5b, aufgezeigt sind dem Verfahren nicht förderlich.

Kraterförmige Erhebungen (42), wie in Abb. 5b dargestellt, sind für das erfindungsgemäße Verfahren zwar förderlich, aber nicht unbedingt notwendig.

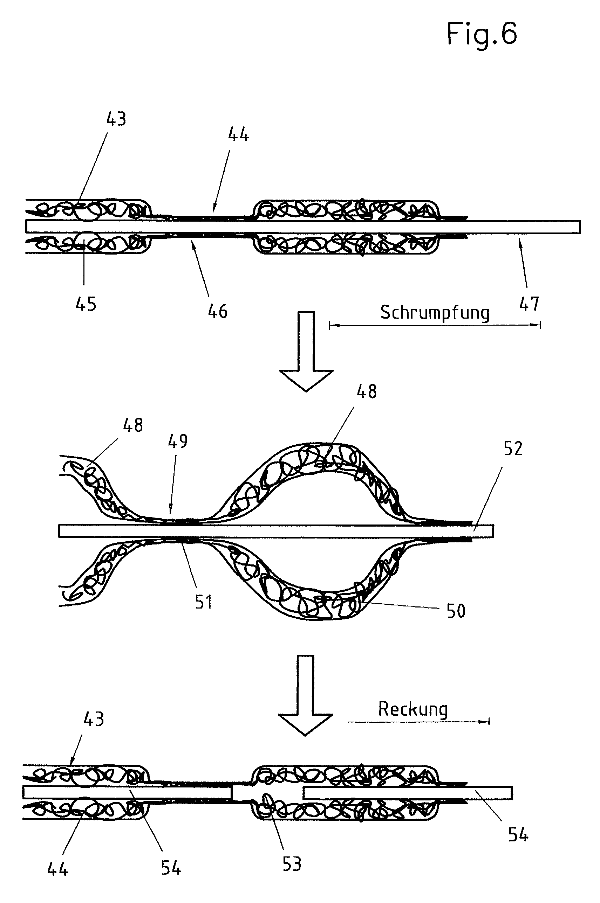

In Abbildung 6 sind die einzelnen Stufen der Bildung von regelmäßigen Folienöffnungen in der Mittelschicht des 3-lagigen Verbundstoffes schematisch dargestellt.

Die Faserflore (43) und (45) werden an den Stellen oben (44) und unten (46) verdichtet und mit der Folie (47) verschweißt. Ein Schrumpf der Folie ist noch nicht eingetreten.

Im zweiten Teil der Abbildung 6 ist die Folie entlang in der Richtung entlang ihres Querschnittes geschrumpft unter entsprechender Längenverkürzung und in dadurch in den Zustand (52) gebracht. Dadurch wird sowohl die Größe der Verschweißstelle als auch der Abstand zwischen den verkleinerten Verschweißstellen (49) oben und (51) unten verkürzt und die Faserflore (43) und (45) in einen gewellten Zustand (48) oben und (50) unten übergeführt. An den Kanten der Verschweißungen (49) und (51) ist die geschrumpfte Folie geschwächt worden. Nach Verdehnung oder Reckung der geschrumpften Ware in einer oder beiden Vorzugsrichtungen (In Abbildung 6 ist nur eine dargestellt) reißt die Folie an der geschwächten Stelle auf und bildet Öffnungen (53), deren Form und Fläche von der Gravur der Walze und dem Schrumpfbetrag abhängt. Die Faserflore (43) und (44) werden nach der Reckung beinahe wieder in den Ausgangszustand zurückversetzt, wenn der gesamte Schrumpfbetrag wieder ausgezogen wird, jedoch mit der Ausnahme, dass die Bindungsflächen in dem verkürzten Zustand (49) und (51) verbleiben.

Für eine Reckung unterhalb des Gesamtschrumpfes werden naturgemäß Wellungen, Erhöhungen oder anderweitige Ausrichtungen in die dritte Dimension verbleiben, die jedoch geringer ausfallen, als in den gewellten Floren (48) und (50) angezeigt. Bei durchgehend linienförmiger Verschweißfläche mit einer Orientierung quer zur Maschinenlaufrichtung, Schrumpf in beiden Richtungen aber Reckung nur in Maschinenlaufrichtung wird eine zusätzlicher Mikrokrepp-Effekt erzeugt, der zusätzlich zur Erhöhung der Textilität der Ware beiträgt.

Der erfindungsgemäße Verbundstoff zeichnet sich durch eine erhöhte Querfestigkeit bei gleichzeitig guter Längsfestigkeit aus und lässt sich insbesondere auf Gebieten einsetzen, die eine hohe mechanische Reißbeanspruchung in beiden Richtungen und zusätzlich Textilität und hohe Atmungsaktivität verlangen. Solche Anwendungen sind Operationskittel, textile Obermaterialien für ein Einmalgebrauch und Gürtel. Durch Verwendung von mit Metall, wie beispielsweise mit Aluminium, Kupfer oder Silber beschichteten Folien, kann zusätzlich zur Festigkeitsverstärkung eine Leitfähigkeit bzw. antimikrobielle Eigenschaften in den neuen Vliesstoffverbund eingebracht werden. Diese zusätzlichen Eigenschaften können sowohl in Hygiene- wie auch Medikalanwendungen genutzt werden, wie z.B. für die Umhüllung bzw. Abdeckung von Windeln oder Damenhygiene-Produkten, bzw. absorbierenden Lagen oder Schichten auf oder in absorbierenden Produkten.

Diese Verwendungen sind ebenfalls Gegenstand der vorliegenden Erfindung.

Die nachfolgenden Beispiele beschreiben die Erfindung ohne diese zu begrenzen.

### Beispiel 1:

Es wurden zwei Stapelfaserflore a und b auf ein Transportband abgelegt. Stapelfaserflor a war aus 100 % gekräuselter, auf 60 mm geschnittener Bikomponentenfaser des Titers 6,7 dtex mit Kern/Mantel-Konfiguration aufgebaut, wies ein Flächengewicht von 28 g/m² auf und wurde wirr in Maschinenlaufrichtung abgelegt. Stapelfaserflor b bestand aus 100% Polypropylen Faser mit einem Titer von 2,8 dtex, einer Schnittlänge von 60 mm und einem Florgewicht von 10 g/m². Er wurde im Gegensatz zu Flor a in Maschinenlaufrichtung abgelegt. Zwischen die beiden Flore wurde eine 17 µm starke, durch das Ringblasverfahren hergestellte biaxial gereckte, transparente PP-Folie quer zur Maschinenlaufrichtung zugeführt und über ein 45° Winkel - Umlenkschwert in Maschinenlaufrichtung umgelenkt und zwischen den beiden Faserfloren a und b positioniert.

Das Komposit aus diesen drei losen Lagen Stapelfaserflor a, PP-Folie und Stapelfaserflor b wurde nach einer vorherigen Verdichtung im Spalt zwischen zwei glatten, nicht beheizten Stahlwalzen komprimiert. Dies erleichterte die Zuführung zu einem Kalander, dessen Hauptelemente aus einer jeweils beheizten glatten und einer gravierten Stahlwalze bestanden. Das Oberflächendesign der gravierten Stahlwalze bestand aus parallel zueinander und beinahe quer zur Maschinenlaufrichtung orientierten Linien, die sich 0,8 mm von dem Walzengrund abhoben und einen Abstand zueinander von 4 min aufwiesen. Der Abstand der Linien zwischen einer Linienkante zur nächsten der folgenden Linie betrug 3 mm und die Breite der Linien oder Stege dementsprechend 1 mm. Dies ergab eine Verschweißfläche von 25 %, bezogen auf Gesamtflächenausdehnung der Ware. Um ausreichende Laufruhe (kein sog. Rattern) bei der thermischen Kalandrierung zu gewährleisten, wurden die Linien nicht exakt in einem Winkel von 90° zur Maschinenlaufrichtung orientiert sondern in einen um 0,8° davon abweichendem Winkel. Außerdem war die garvierte Kalanderwalze mit sog. Stützrändern versehen zur zusätzlichen Erhöhung der Laufruhe.

Die drei Lagen wurden mit einer Geschwindigkeit von 3 m/min. durch den Kalanderpress-Spalt durchgeführt und dabei miteinander verschweißt. Die Temperatur der beiden Kalanderwalzen betrug jeweils 145°C und die Kalanderlinienkraft 55 N/mm. Die Faserflorlage a mit den grobtitrigen 6,7 dtex-Fasern war der Gravurwalze zugewandt. Nach Verlassen des Walzenpressspaltes umschlang die Ware in etwa ein Viertel der Glattwalzenoberfläche bevor sie dann weitergeleitet wurde. Durch die dadurch erzielte relativ lange Verweilzeit und den Temperaturkontakt erfuhr die Folie in der Mitte des Verbundstoffes einen Flächenschrumpf in beiden Richtungen. Durch Umlenkung über eine gebogene Walze (einen sogenannten krummer Hund) wurde ein Teil des Schrumpfes quer zur Maschinenlaufrichtung wieder herausgezogen.

Durch Beibehaltung derselben Geschwindigkeit vor und nach dem Kalanderpressspalt wurde nach dem Kalanderpressspalt eine starke Zugspannung ausgeübt, die im noch warmen Zustand der Ware auf der Kalanderglattwalze zu einem Aufreißen der zentral positionierten Folie zu Folienstreifen führte. Es wurde damit eine Kompositstruktur mit sich ständig wiederholenden 3 unterschiedlichen Zonen erzeugt.
1. Eine lineare, matt aussehende Verschweißzone mit einer Breite von ca. 0,9 mm.
2. Eine lineare Zone, bei der die beiden Vlieslagen mit der Folie nicht verschweißt waren, in einer Breite von ca. 1,4 mm. Diese Folienstreifenbereiche hatten ihren Glanz beibehalten.
3. Eine lineare Zone, die nur aus den beiden Vlieslagen bestand, in einer Breite von ca. 1,6 mm und somit hochdurchlässig war.

Die folienfreie, durchlässige Zone betrug somit 1,6/3,9 = 41,0 %, bezogen auf die Gesamtfläche des Verbundstoffes. Die Seite des Faserflors a bildete schwache Wellen, wahrend die Feinflorseite b aufgrund ihres Kontaktes mit der Glattwalze flach abgeglättet war.

Die ursprüngliche Summe des Materialeinsatzes an Faserfloren und Folie pro m² Fläche vor der Kalanderbehandlung betrug 28 + 15 + 8 = 51 g/m² (ohne Schrumpf).

Das Fertigmaterialgewicht nach Anwendung der Schrumpf-/Reckbedingungen betrugt dagegen 58 g/m². Die Verkürzung der Verschweißlinienbreite von 1 mm auf 0,90 mm ergab einen Flächenverlust um 0,1/4,0 x 100 = 2,5%. Durch den nicht vollständig quer zur Maschinenlaufrichtung herausgezogenen Schrumpf über gebogene Walzen verblieb in Querrichtung eine Kürzung um 9,8%. Dieser verbleibende Querschrumpf äußerte sich in einer Mikrokreppung in Maschinenlaufrichtung und verlieh diesem Anwendungsbeispiel eine hohe Textilität und Drapierfähigkeit in beiden Richtungen.

Die in Beispiel 1 beschriebene Variante der Erfindung könnte beispielsweise als Schlingenkomponente von mechanischen Verschlusssystemen eingesetzt werden, insbesondere, wenn hohe Querfestigkeiten gefordert werden. Die Schlingenseite ist die Grobfaserflorseite a.

### Vergleichsbeispiel zu 1:

Es wurden die beiden Stapelfaserflore unter Weglassen der 17 Mikrometer PP-Schrumpffolie unter den Bedingungen wie bei Beispiel 1 angegeben. Dadurch wurde ohne jeglichen Schrumpf ein 2-lagiger Vliesverbundstoff mit einem Flächengewicht von 38 g/m² erzeugt.

In Tabelle a sind die wichtigsten Prüfergebnisse der Beispieles 1 und des Vergleichsbeispieles zu 1 wiedergegeben

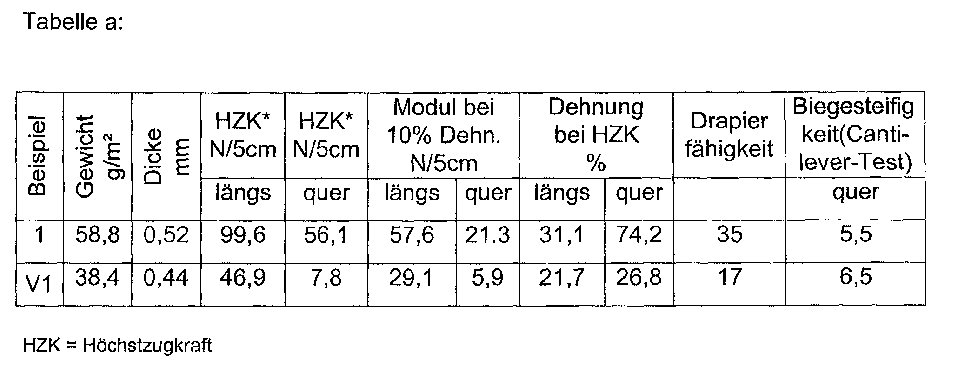

Die Drapierfähigkeit wurde nach DIN 54 306 bestimmt. Hierzu wurde jeweils der kreisrund ausgestanzte Probekörper mit der flacheren Seite nach unten auf den Mess-Teller aufgelegt. Die flachere Seite des Verbundstoffes ist diejenige Seite, die bei der Kalandrierung Kontakt zur Glattwalze hat.

Die Biegesteifigkeit wurde nach DIN 53 362 nach der sogenannten Cantilever-Methode bestimmt. Die Streifern für die Messung wurden jeweils nur quer zur Maschinenlaufrichtung ausgestanzt und in die Maßapparatur so eingespannt, dass jeweils die glatte Seite des Verbundstoffes nach unten gerichtet war.

### Beispiel 2:

Im Vergleich zu Beispiel 1 wurde im Beispiel 2 zwar die gleicher Versuchsanordnung gewählt, es wurden jedoch eine 15 µm, durch das Blasverfahren biaxial gereckte PP-Schrumpffolie eingesetzt und der Wirrflor a des Beispieles 1 wurde durch einen nur 28 g/m² schweren Längsflor mit der Faserzusammensetzung 70% Polyesterfaser (PET) mit einem Titer von 6,7 dtex und einer Schnittlänge von 60 mm sowie 30% Kern-/Mantel Bikomponentenfaser aus Polyethylenterephtalat und Polypropylen mit einem Titer von 3,3 dtex und einer Schnittlänge von 51 mm verwendet. Die andere Seite der Folie wurde mit einem 10 g/ g/m² schweren Stapelfaserflor mit der Faserzusammensetzung 70% Polyesterfaser (PET) mit einem Titer von 1,7 dtex und einer Schnittlänge von 38 mm sowie 30% Kern-/Mantel-Bikomponentenfaser mit einem Titer von 3,3 dtex und einer Schnittlänge von 51 mm unterlegt.

Die Glattwalzentemperatur wurde auf 148°C und die Gravurwalzentemperatur auf 151°C eingestellt. Die Linienkraft im Pressspalt betrug wieder 55 N/mm. Die Geschwindigkeit lag bei 4,5 m/min. Unter den wie in Beispiel 1 beschrieben Verhältnissen wurden die annähernd gleichen Schrümpfe und linienförmigen Folienstreifenbildung in der Mitte des Verbundstoffes erzielt.

Vergleichsbeispiel zu 2: Wie im Vergleichbeispiel zu 1 wurde auch hier die 15 µm PP-Folie weggelassen und ein "linear seal" gebundes Komposit unter den Kalanderbedingungen, wie im Beispiel 2 beschrieben, hergestellt.

Die Prüfergebnisse des Beispieles 2 und des Vergleichsbeispieles zu 2 sind in der folgenden Tabelle b wiedergegeben:

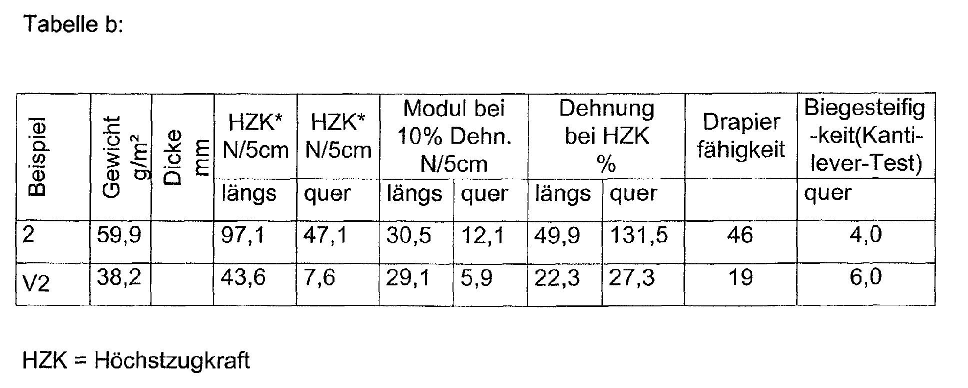

Man sieht aus den Ergebnissen der Tabelle b noch deutlicher als in Tabelle a den Anstieg der HZK quer um das mehr als 6-fache verbunden mit einer extrem starken Steigerung der Dehnung quer auf das ca. 5-fache, was auf die relativ hohe Verdehnbarkeit der gebildeten Folienquerbändchen zurückzuführen ist.

### Beispiel 3:

Mit derselben Anordnung wie in Beispiel 1 und 2 wurde ein 25 g/m² schwerer Wirrflor aus 100 % Polypropylen-Stapelfasern mit einem Titer von 1,7 und einer Stapelfaserlänge von 40 mm in Maschinenlaufrichtung und ein 8 g/ g/m² schwerer Längsflor aus 100% Polypropylenfasern mit einem Titer von 1,7 dtex einer Schnittlänge von 40 mm auf Transportbändern abgelegt und zwischen den beiden Floren wieder eine PP-Folie in der im Beispiel 1 beschriebenen Art und Weise positioniert. Die im Beispiel 3 eingesetzte Polypropylen-Folie unterschied sich insbesondere von den beiden in Beispiel 1 und 2 verwendeten Folien dadurch, dass sie nach dem Chill-Roll-Verfahren (d.h. Aufgießen einer Folie aus einer Breitschlitzdüse auf eine Walze) hergestellt worden ist und gezielt extrem stark mit einem Reckverhältnis von 1 : 5 in Maschinenlaufrichtung (über mehrere Reckzonen und Walzen) und quer zur Maschinenlaufrichtung auf das 9-fache der ursprünglichen Breite gereckt worden war (Reckverhältnis quer 1 : 9). Die Stärke dieser extrem stark biaxial gereckten Polypropylen-Folie betrug nur 12 µm. Der Schmelzpunkt der Folie lag im Bereich von 160 bis 166°C und ihre Höchstzugkräfte längs bei 83 N/5cm und quer von 150 N/5cm.

Die Temperaturen beider Kalanderwalze betrugen 155°C und der Liniendruck im Kalanderpressspalt wiederum 55 N/mm.

Wie in Beispiel 1 wurde durch Anlegen einer Zugspannung linienförmige Folienstreifen in der Mitte des Verbundstoffes erzielt. In der Ware verblieb nach Entlastung eine Längenkürzung um 2.6 % und eine im Vergleich zu Beispiel 1 deutlich höheren Breitenverlust durch Schrumpf von 15.4%. der starke Breitenverlust zeigt sich in einem starken Mikrokreppeffekt über die breite, d. h. entlang der Folienstreifen. Der Gesamtflächenverlust betrug damit 17,6%

Es wurde in Beispiel 3 ein Verbundstoff geschaffen, der optisch beinahe dem mit Beispiel 1 identisch war, sich jedoch von Beispiel 1 insbesondere durch höhere Querfestigkeiten unterschied und einen etwas härten Griff. Die hohe Steigerung der HZK in Querrichtung gegenüber Beispiel 1 war ausschließlich auf den hohen Reckgrad der PP-Folie quer zur Maschinenlaufrichtung (Reckgrad 1 : 9) zurückzuführen.

Vergleichsbeispiel zu 3: Es wurde, wie bereits bei der Vergleichbeispielen zu 1 und 2 beschrieben, die Folie weggelassen, d.h. unter den ansonsten in Beispiel 3 beschriebenen Verhältnissen ein 2-schichtiger Vliesstoff mit starker Längsorientierung hergestellt.

Die Prüfdaten des Beispieles 3 und des Vergleichbeispieles zu 3 sind in Tabelle c zusammengefasst:

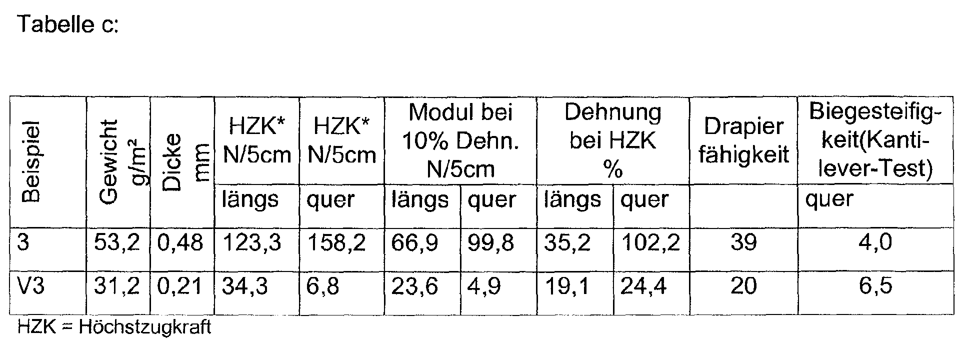

Aus Tabelle c wird sehr klar ersichtlich, wie stark insbesondere die HZK quer durch den erfindungsgemäßen Aufbau und das angewandte Verfahren zur Herstellung desselben angehoben werden kann. Das Längs-/Quer-Verhältnis von 5 / 1 im Vergleichsmuster konnte in Beispiel drei auf 1 / 1,28 völlig verändert werden; d.h. aus einem stark längsorientierten Vliesstoff wurde sogar ein Verbundstoff mit höheren Quer- als Längsfestigkeiten geschaffen.

## Patentansprüche

1. Verbundstoff umfassend mindestens einen Vliesstoff und ein damit thermisch verbundenes, in Form von regelmäßig wiederkehrenden Mustern angeordnetes Verstärkungsmaterial, das sich von einer Folie aus thermoplastischem Material ableitet.

2. Verbundstoff nach Anspruch 1, **dadurch gekennzeichnet, dass** dieser mindestens drei Schichten in der Abfolge Vliesstoff / Verstärkungsmaterial / Vliesstoff aufweist.

3. Verbundstoff nach Anspruch 1, **dadurch gekennzeichnet, dass** der Vliesstoff ein Stapelfaservliesstoff ist.

4. Verbundstoff nach Anspruch 1, **dadurch gekennzeichnet, dass** die Folie aus thermoplastischem Material eine uni- oder biaxial gereckte Folie ist, die vorzugsweise aus Polyolefinen besteht.

5. Verbundstoff nach Anspruch 4, **dadurch gekennzeichnet, dass** die Folie aus Polyethylen oder insbesondere aus Polypropylen besteht.

6. Verbundstoff nach Anspruch 1, **dadurch gekennzeichnet, dass** der Vliesstoff mit der Folie durch thermisches Verschweißen mit mindestens einer Oberfläche des Vliesstoffs in der Form von regelmäßig wiederkehrenden Mustern verbunden ist, und die Folie an diesen Stellen der Oberfläche des Vliesstoffs erhalten geblieben ist und an anderen Stellen der Oberfläche des Vliesstoffs infolge Zerreißens der Folie nicht mehr vorliegt.

7. Verbundstoff nach Anspruch 6, **dadurch gekennzeichnet, dass** das regelmäßig wiederkehrende Muster die Form von regelmäßig senkrecht zur und/oder in Maschinenlaufrichtung angeordneten Linien oder Balken aufweist.

8. Verbundstoff nach Anspruch 6, **dadurch gekennzeichnet, dass** das regelmäßig wiederkehrende Muster die Form von regelmäßig angeordneten Linien in der Form von Hexagons aufweist.

9. Verbundstoff nach Anspruch 6, **dadurch gekennzeichnet, dass** das dieser sich rhythmisch wiederholende, unterschiedliche drei Bereiche aufweist, welche Folienstreifenbereiche (4) sind, die mit den Fasern des Vliesstoffes verschweißt sind, Folienstreifenbereiche (3) sind, die mit den Fasern des Vliesstoffes nicht verschweißt sind und die Bereiche (8) sind, die in diesem Bereich ausschließlich aus ungebundenen Fasern bestehen.

10. Verbundstoff nach Anspruch 2, **dadurch gekennzeichnet, dass** das der eine Vliesstoff aus gröberen Fasern, die ein sehr gutes Wiedererholvermögen aufweisen, besteht, und dass der zweite Vliesstoff aus feineren Fasern mit einem niedrigen Wiedererholvermögen besteht.

11. Verbundstoff nach Anspruch 1, **dadurch gekennzeichnet, dass** dieser ein Flächengewicht von 15 bis 250 g/m² aufweist.

12. Verbundstoff nach Anspruch 1, **dadurch gekennzeichnet, dass** dieser im wesentlichen eine flächige Struktur und im wesentlichen keine in Bezug auf die Flächenebene alternierend auftretende Erhebungen und Vertiefungen aufweist.

13. Verbundstoff nach Anspruch 9, **dadurch gekennzeichnet, dass** die Folienstreifenbereiche unbeschichtet sind.

14. Verbundstoff nach Anspruch 9, **dadurch gekennzeichnet, dass** die Folienstreifenbereiche zumindest einseitig metallbeschichtet sind.

15. Verbundstoff nach Anspruch 9, **dadurch gekennzeichnet, dass** die Folienstreifenbereiche zumindest einseitig beschichtet sind und dem Verbundstoff eine Leitfähigkeit und/oder antimikrobielle Wirksamkeit verleihen.

16. Verfahren zur Herstellung eines Verbundstoffs nach Anspruch 1 umfassend die Maßnahmen:
a) Kombination von mindestens einem Flor mit einer schrumpffähigen Folie aus thermoplastischem Material,
b) thermisches Verschweißen zwischen dem Flor und der schrumpffähigen Folie in Form eines regelmäßig wiederkehrenden Musters,
c) Erhitzen des erhaltenen Verbundes während oder nach Schritt b) auf eine solche Temperatur, dass sich der Vliesstoff ausbildet, der Schrumpf der schrumpffähigen Folie ausgelöst wird und sich regelmäßig in Bezug auf die Flächenebene alternierend auftretende Erhebungen und Vertiefungen ausbilden,
d) Verstrecken des Verbundes zumindest in einer Richtung, so dass die Erhebungen und Vertiefungen im wesentlichen verschwinden und die Folie an den nicht mit dem Vliesstoff verschweißten Stellen reißt.

17. Verfahren nach Anspruch 16, **dadurch gekennzeichnet, dass** das thermische Verschweißen zwischen Flor und schrumpffähiger Folie durch Durchleiten des Verbundes durch einen Kalander erfolgt, der mindestens eine glatte und eine gravierte Walze aufweist.

18. Verfahren nach Anspruch 17, **dadurch gekennzeichnet, dass** die gravierte Walze regelmäßig wiederkehrende Muster in Form von ununterbrochenen Erhebungen aufweist, die sich nach der Kalandrierung als Vertiefungen in dem Verbundstoff abbilden.

19. Verfahren nach Anspruch 18, **dadurch gekennzeichnet, dass** die ununterbrochenen Erhebungen der gravierten Walze gerade oder gekrümmte bzw. gebogene Linien sind, die parallel zueinander ausgerichtet sind.

20. Verfahren nach Anspruch 17, **dadurch gekennzeichnet, dass** die gravierte Walze aus mindestens zwei Serien von ununterbrochenen, regelmäßig wiederkehrenden und parallel zueinander ausgerichteten Erhebungen besteht, die zueinander einen Winkel bilden.

21. Verfahren nach Anspruch 18, **dadurch gekennzeichnet, dass** die regelmäßig wiederkehrenden Muster in Form von regelmäßig angeordneten Linien in der Form von Hexagons ausgebildet sind.

22. Verwendung des Verbundstoffs nach Anspruch 1 als Operationskittel, textile Obermaterialien für ein Einmalgebrauch und Gürtel.

23. Verwendung des Verbundstoffs nach Anspruch 1 im Hygiene- und/oder Medikalbereich, insbesondere für die Umhüllung bzw. Abdeckung von absorbierenden Produkten.

24. Verwendung nach Anspruch 23, **dadurch gekennzeichnet, dass** die absorbierenden Produkte Windeln oder Damenhygiene-Produkte sind.
